(19) Europäisches Patentamt
European Patent Office
Office européen des brevets

(11) EP 4 635 968 A2

(12) EUROPEAN PATENT APPLICATION
published in accordance with Art. 153(4) EPC

(43) Date of publication:
**22.10.2025 Bulletin 2025/43**

(21) Application number: **24744436.7**

(22) Date of filing: **19.03.2024**

(51) International Patent Classification (IPC):
***C07J 41/00*** *(2006.01)* ***A61K 31/57*** *(2006.01)*
***A61P 25/24*** *(2006.01)* ***A61P 25/22*** *(2006.01)*
***A61P 25/08*** *(2006.01)* ***A61P 25/06*** *(2006.01)*
***A61P 25/20*** *(2006.01)* ***A61P 25/00*** *(2006.01)*
***A61P 25/28*** *(2006.01)* ***A61P 25/16*** *(2006.01)*
***A61P 15/00*** *(2006.01)*

(86) International application number:
**PCT/CN2024/082534**

(87) International publication number:
**WO 2024/153267 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.01.2023 CN 202310096207**

(71) Applicant: **Hunan Mingrui Pharmaceutical Co., Ltd.**
**Changsha, Hunan 410300 (CN)**

(72) Inventors:
- **AN, Ming**
  **Beijing 100023 (CN)**
- **ZHANG, Xu**
  **Beijing 100023 (CN)**
- **GAO, Zheng**
  **Beijing 100023 (CN)**
- **KUANG, Bin**
  **Beijing 100023 (CN)**
- **LV, Ning**
  **Beijing 100023 (CN)**
- **HOU, Huifang**
  **Beijing 100023 (CN)**

(74) Representative: **Pons IP**
**Glorieta Rubén Darío 4**
**28010 Madrid (ES)**

Remarks:
The restoration of the right of priority granted by the receiving Office is effective before the EPO (R. 49ter.1(a) PCT)

# (54) NEUROSTEROID DERIVATIVES AND USE THEREOF

(57) Provided in the present invention are a neurosteroid derivative having a structure as represented by formula (I) and the use thereof. Compared with the prior art, the neurosteroid derivative having the structure as represented by formula (I) provided in the present invention has a significantly higher bioavailability, and can be administered by means of a non-intravenous administration route for use in the treatment and/or prevention of GABA-A receptor-mediated diseases.

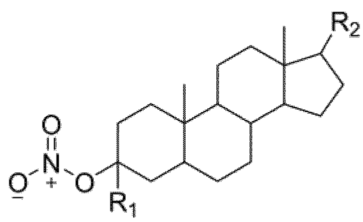

(I)

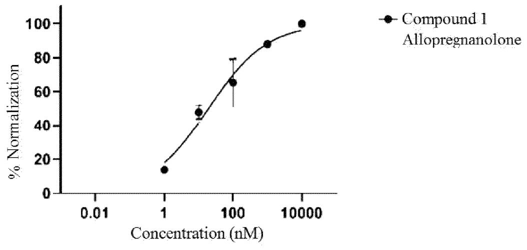


| | Compound 1 3μM | Allopregnanolone3μM |
|---|---|---|
| HillSlope | 0.5627 | 0.2059 |
| EC50 | 10.514 | 8.328 |

FIG. 1

## Description

## TECHNICAL FIELD

The present disclosure relates to the field of pharmaceutical chemistry, in particular to a class of neurosteroid derivatives and medical use thereof.

## BACKGROUND

GABA (γ-aminobutyric acid), a major neurotransmitter in the central nervous system, can increase the flow of ions inside and outside the nerve cell membrane by mediating downstream ionotropic channel GABA-A receptors, thereby exerting physiological efficacy. Active sites of GABA-A receptors can bind to GABA, as well as many drugs such as muscimol, gaboxadol, and bicuculline. There are a number of isomeric modulators that can indirectly modulate GABA-A receptor activity, including benzodiazepines, barbiturates, and inhaled anesthetics.

Neurosteroids are steroid hormone derivatives that are devoid of hormonal effects, but still affect neuronal excitability through modulation of ionotropic receptors, and can bind to GABA-A receptors. Dehydroepiandrosterone, progesterone, and their metabolites can modulate the expression of GABA-A receptor subunits through genetic mechanisms, thereby affecting neurotransmitters. Neurosteroids exhibit fewer subunit restrictions and exhibit a wider application range than benzodiazepines. Neurosteroid drugs targeting GABA-A receptors currently in development and/or under development include allopregnanolone, ganaxolone, alfaxalone, and alfadolone.

Allopregnanolone, also known as 5α-pregnan-3α-ol-20-one, is an endogenous inhibitory pregnane neurosteroid. Allopregnanolone has effects similar to those of other positive allosteric modulators of the GABA actions at GABA-A receptors, such as benzodiazepines. Endogenously produced allopregnanolone plays a key neurophysiological role by fine-tuning GABA-A receptors and modulating the effects of several positive allosteric modulators and agonists at GABA-A receptors.

Ganaxolone, also known as 3α-hydroxy-3-methyl-5α-pregnan-20-one, is a synthetic neurosteroid analog that acts as a positive allosteric modulator of GABA-A receptors. Ganaxolone has shown promise for the treatment of temporal lobe epilepsy seizures, as well as catamenial epilepsy. Ganaxolone is also under study for the treatment of post-traumatic stress disorder, Fragile-X syndrome, neurological headache, neonatal seizure and post-natal depression. Ganaxolone has been approved by the US FDA for the treatment of protocadherin-19 gene (PCDH19) female epilepsy, and is well-tolerated in both adults and children.

Positive allosteric modulators of GABA-A receptors, including allopregnanolone and ganaxolone, have defects such as low water solubility and metabolic instability, resulting in insufficient bioavailability of drugs in the human body, which brings limitations to clinical application. Currently, structural modifications to positive allosteric modulators of GABA-A receptors, including allopregnanolone and ganaxolone, primarily focus on improving water solubility to promote drug absorption and improve bioavailability and pharmacokinetic properties. For example, CN 108148106A discloses a class of water-soluble allopregnanolone derivatives and use thereof, where the water-soluble allopregnanolone derivatives have good physical/chemical stability and good water solubility, and can rapidly decompose in plasma to release the active drug (allopregnanolone), thereby quickly producing pharmacological effects. Alternatively, structural modifications have been made to positive allosteric modulators of GABA-A receptors to reduce their metabolic rate, thereby achieving oral administration. For example, CN 109503694A discloses a novel GABA-A receptor modulator, in which the introduction of aryl or heteroaryl adjacent to the 20-carbonyl of allopregnanolone reduces the metabolic rate of the compound while maintaining good biological activity, thereby achieving oral administration.

Currently, there is an urgent clinical need for GABA-A receptor modulators with good bioavailability, in particular those with stable *in vivo* metabolism. An object of the present disclosure is to find drugs with better *in vivo* bioavailability, in particular those with better *in vivo* metabolic stability, by structural modifications of existing neurosteroid drugs.

## SUMMARY

In one aspect, the present disclosure provides a compound having a structure as represented by formula (I):

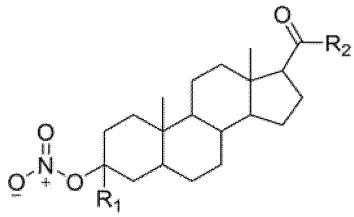

(I)

or a stereoisomer, a tautomer, an isotope-enriched analog, a solvate, and a pharmaceutically acceptable salt thereof, where $R_1$ and $R_2$ are as defined herein.

In one aspect, the present disclosure provides a method for preparing the compound having the structure as represented by formula (I) or the stereoisomer, the tautomer, the isotope-enriched analog, the solvate, and the pharmaceutically acceptable salt thereof.

In another aspect, the present disclosure provides a pharmaceutical composition for treating and/or preventing central nervous system diseases, including the compound having the structure as represented by formula (I) or the stereoisomer, the tautomer, the isotope-enriched analog, the solvate, the pharmaceutically acceptable salt thereof, and a pharmaceutically acceptable carrier.

In another aspect, the present disclosure further provides use of the compound having the structure as represented by formula (I) or the stereoisomer, the tautomer, the isotope-enriched analog, the solvate, and the pharmaceutically acceptable salt thereof in manufacture of a medicament for treating and/or preventing central nervous system diseases.

In a further aspect, the present disclosure further provides a method for treating and/or preventing central nervous system diseases, including: administering to an individual in need the compound having the structure as represented by formula (I) or the stereoisomer, the tautomer, isotope-enriched the analog, the solvate, and the pharmaceutically acceptable salt thereof in a therapeutically effective amount.

Beneficial effects:

Compared with the prior art, the compound or the stereoisomer, tautomer, isotope-enriched analog, solvate, and pharmaceutically acceptable salt thereof of some embodiments of the present disclosure exhibits significantly better pharmacokinetic properties, in particular *in vivo* metabolic stability, and can be administered by means of a non-intravenous administration route for treating a variety of central nervous system diseases.

## BRIEF DESCRIPTION OF THE DRAWINGS

The drawings, which are intended to provide a further understanding of the present disclosure and constitute a part of this description, are intended to explain the present disclosure together with the examples of the present disclosure and do not constitute a limitation on the present disclosure.

FIG. 1 shows the modulatory effect of compound 1 of the present disclosure on the GABA-A ($\alpha4\beta3\delta$) receptor.

## DETAILED DESCRIPTION OF THE EMBODIMENTS

### Definitions

As used in the description, the following terms and phrases are generally intended to have the meanings set forth below, unless the context in which they are used indicates other meanings.

As used herein, the term "alkyl" refers to a monovalent group of a branched or unbranched saturated hydrocarbon chain having 1 to 12 carbon atoms (more typically having 1 to 10 carbon atoms, 1 to 8 carbon atoms, or 1 to 6 carbon atoms). Representative examples of the term include groups such as methyl, ethyl, 1-propyl(n-propyl), 2-propyl(isopropyl), 1-butyl(n-butyl), 2-methyl-1-propyl(isobutyl), 2-butyl(sec-butyl), 2-methyl-2-propyl(tert-butyl), 1-pentyl(n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, 1-octyl, 1-nonyl, and 1-decyl.

As used herein, the term "alkenyl" refers to a monovalent linear or branched unsaturated hydrocarbon group having the indicated number of carbon atoms (e.g., having 2 to 12 carbon atoms, 2 to 10 carbon atoms, 2 to 8 carbon atoms, or 2 to 6 carbon atoms) and having carbon-carbon double bonds (e.g., 1, 2, or 3 carbon-carbon double bonds). In some

embodiments of the present disclosure, examples of alkenyl include, but are not limited to vinyl (i.e., $-CH=CH_2$), propen-1-yl (i.e., $-CH=CHCH_3$), propen-3-yl (or allyl, i.e., $-CH_2CH=CH_2$), propen-2-yl (i.e., $-C(CH_3)=CH_2$), and butadienyl (including 1,2-butadienyl and 1,3-butadienyl).

As used herein, the term "alkynyl" refers to a monovalent linear or branched unsaturated hydrocarbon group having the indicated number of carbon atoms (e.g., having 2 to 12 carbon atoms, 2 to 10 carbon atoms, 2 to 8 carbon atoms, or 2 to 6 carbon atoms) and having carbon-carbon triple bonds (e.g., 1, 2 or 3 carbon-carbon triple bonds). In some embodiments of the present disclosure, examples of alkynyl include, but are not limited to, ethynyl (i.e., $-C\equiv CH$), propargyl (i.e., $-CH_2C\equiv CH$), and propynyl (i.e., $-C\equiv CCH_3$).

As used herein, the term "cycloalkyl" refers to a monovalent saturated carbocyclic group that has a monocyclic ring or multiple fused or bridged rings and that has 3 to 12 carbon atoms (more typically having 3 to 10 carbon atoms, 3 to 8 carbon atoms, or 3 to 6 carbon atoms). In some embodiments, cycloalkyl groups include monocyclic structures (e.g., cyclopropyl, cyclobutyl, cyclopentyl, cyclooctyl), or polycyclic structures (e.g., adamantyl and bicyclo[2.2.1]heptanyl), or cycloalkyl groups fused to aryl groups (e.g., indane), where the point of attachment is through the cycloalkyl group.

As used herein, the term "aryl" refers to an aromatic carbocyclic group that has a monocyclic ring (e.g., phenyl) or a polycyclic ring (e.g., biphenyl) or multiple fused (condensed) rings (e.g., naphthyl, fluorenyl, and anthryl) and that has 6 to 14 carbon atoms (more typically having 6 to 10 carbon atoms, or 6 carbon atoms). Representative examples of the term include groups such as phenyl, fluorenyl, naphthyl, anthracenyl, and 1,2,3,4-tetrahydronaphthalene (if the point of attachment is through an aryl group).

As used herein, the term "heteroaryl" refers to an aromatic ring group having a monocyclic ring or a fused (condensed) polycyclic ring (e.g., including 2 or 3 rings), where the ring contains 5 to 14 ring atoms, and the ring atoms include, in addition to the carbon atoms, at least one more heteroatoms selected from the group consisting of oxygen, nitrogen and/or sulfur. If the ring is aromatic, the sulfur and nitrogen atoms may also be exist in oxidized form. A fused (condensed) polycyclic heteroaryl is formed by fusing the monocyclic heteroaryl as defined above with one or more rings selected from the group consisting of the following to form a fused polycyclic system: heteroaryl (to form, for example, naphthyridinyl, such as 1,8-naphthyridinyl), heterocycle (to form, for example, 1,2,3,4-tetrahydronaphthyridinyl, such as 1,2,3,4-tetrahydro-1,8-naphthyridinyl), carbocyclic (to form, for example, 5,6,7,8-tetrahydroquinolinyl) and aryl (to form, for example, indazolyl). Such a fused polycyclic system may optionally be substituted with one or more (such as 1, 2, 3 or 4) oxo groups on the carbocyclic or heterocyclic moiety of the fused ring. When valency permits, the rings of the fused polycyclic system may be interconnected via fusion, spiro ring and bridging bonds. It should be understood that the individual rings of the fused polycyclic system may be connected in any order relative to each other. It should also be understood that the point of attachment of the fused polycyclic system may be at any position within the fused polycyclic system, including the heteroaryl, heterocyclic, aryl or carbocyclic moiety of the fused polycyclic system. It should also be understood that the point of attachment of the heteroaryl may be at any suitable atom of the heteroaryl, including carbon atoms and heteroatoms (such as nitrogen). Exemplary heteroaryl includes, but is not limited to: pyridyl, pyrrolyl, pyrazinyl, pyrimidinyl, pyridazinyl, pyrazolyl, thienyl, indolyl, imidazolyl, oxazolyl, isoxazolyl, thiazolyl, furyl, oxadiazolyl, thiadiazolyl, quinolinyl, isoquinolinyl, benzothiazolyl, benzoxazolyl, indazolyl, quinoxalinyl, quinazolinyl, 5,6,7,8-tetrahydroisoquinolinyl, benzofuryl, benzimidazolyl, thianaphthyl, pyrrolo[2,3-b]pyridyl, quinazolin-4(3H)-one, triazolyl, 4,5,6,7-tetrahydro-1H-indazolyl and 3b,4,4a,5-tetrahydro-1H-cyclopropa[3,4]cyclopenta[1,2-c]pyrazolyl.

As used herein, the term "heterocyclyl" refers to a monovalent saturated or partially unsaturated group having a 3- to 8-membered monocyclic ring or multiple fused (condensed) or bridged rings, where the ring has 3 to 14 ring atoms, and the ring atoms include, in addition to the carbon atoms, at least one or more heteroatoms selected from the group consisting of oxygen, nitrogen and/or sulfur. Examples of heterocyclyl include, but are not limited to, tetrahydrofuranyl, morpholinyl, piperidyl, piperazinyl, dihydropyridyl, 4,5,6,7-tetrahydro-1H-benzo[d]imidazolyl, benzo[d]imidazolyl, 4,5,6,7-tetrahydro-1H-imidazo[4,5-c]pyridyl, aziridinyl, azetidinyl, tetrahydropyrrolyl, azepanyl, azocanyl, oxiranyl, oxetanyl, tetrahydrofuryl, tetrahydropyranyl, oxepanyl, oxocanyl, thiiranyl, thietanyl, tetrahydrothienyl, tetrahydrothiopyranyl, thiepanyl, thiocanyl, tetrahydroimidazolyl, tetrahydropyrazolyl, tetrahydrooxazolyl, tetrahydroisoxazolyl, tetrahydrothiazolyl, tetrahydroisothiazolyl, dioxanyl, thioxanyl, and dithianyl.

As used herein, the term "halogen" refers to fluorine, chlorine, bromine, and iodine.

As used herein, the term "alkoxy" refers to an "alkyl-O-" group, where the alkyl is as defined herein. Representative examples of the term include groups such as methoxy, ethoxy, n-propoxy, isopropoxy, n-butoxy, isobutoxy, and tert-butoxy.

As used herein, the term "therapeutically effective amount" refers to an amount sufficient to affect treatment, as defined below, when administered to a mammal in need of such treatment. The therapeutically effective amount will vary depending on the subject and disease condition being treated, the weight and age of the subject, the severity of the disease condition, the mode of administration, etc., which can be readily determined by a person of ordinary skill in the art.

As used herein, the term "stereoisomer" refers to compounds that have the same chemical composition and connectivity, but differ in the orientation of the atoms in space, where the orientation cannot be interchanged by single bond rotation. The term "stereoisomer" includes "diastereomer" and "enantiomer". The term "diastereomer" refers to a

stereoisomer that has two or more chiral centers and whose molecules are not mirror images of each other. Diastereomers have different physical properties, such as melting point, boiling point, spectral properties, and reactivity. Mixtures of diastereomers can be separated by high-resolution analytical procedures such as crystallization, electrophoresis, and chromatography. The term "enantiomer" refers to two stereoisomers of a compound that are non-superimposable mirror images of each other.

As used herein, the term "tautomer" refers to two (or more) compounds that coexist and differ from each other only in the position of one (or more) mobile atom(s) and the distribution of electrons, such as keto-enol tautomers.

As used herein, the term "pharmaceutically acceptable" means that a substance or composition must be chemically and/or toxicologically compatible with the other ingredients containing the preparation and/or the mammal treated therewith.

Any general formula or structure given herein, including general formula I or any general formula disclosed herein, is also intended to represent unlabeled forms and isotope-labeled forms of the compounds. Such forms of the compounds may also be referred to as "isotope labels" or "isotope-enriched analogs". Isotopically labeled compounds have the structure depicted herein, except that one or more atoms are replaced with atoms having a selected atomic mass or mass number. Examples of isotopes that can be incorporated into the compound of the present disclosure include isotopes of hydrogen, carbon, nitrogen, oxygen, phosphorus, fluorine, chlorine, and iodine, such as, but not limited to, $^{2}H$ (deuterium, D), $^{3}H$ (tritium), $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{36}Cl$, $^{123}I$, and $^{125}I$. Such compounds are synthesized by means well known in the art, for example by using starting materials in which one or more hydrogens have been replaced with deuterium.

As used herein, the term "solvate" refers to an association compound or complex of one or more solvent molecules with a compound of the present disclosure. Examples of solvents that form solvates include, but are not limited to, water, isopropanol, ethanol, methanol, dimethylsulfoxide (DMSO), ethyl acetate, acetic acid, and ethanolamine. The term "hydrate" refers to a complex in which the solvent molecule is water.

## Compound

In one embodiment, the present disclosure provides a compound having a structure as represented by formula (I),

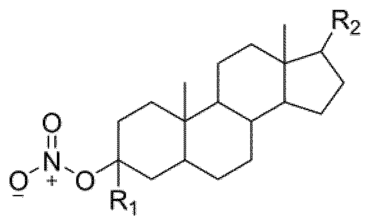

(I)

or a stereoisomer, tautomer, an isotope-enriched analog, a solvate, and a pharmaceutically acceptable salt thereof, where

$R_1$ is selected from the group consisting of hydrogen and $C_1$-$C_{12}$ alkyl; and

$R_2$ is selected from the group consisting of R-(C=O)-, R-(C=S)-, R-(S=O)-, R-($SO_2$)-, R-CH(OH)-, R-O-, R-S-, and $NO_2$, where R is selected from the group consisting of hydrogen, $C_1$-$C_{12}$ alkyl, $C_2$-$C_{12}$ alkenyl, $C_2$-$C_{12}$ alkynyl, $C_3$-$C_{12}$ cycloalkyl, $C_6$-$C_{14}$ aryl, heteroaryl, heterocyclyl, and $NR_3R_3'$, where the alkyl, alkenyl, alkynyl, cycloalkyl, aryl, heteroaryl, and heterocyclyl are optionally substituted with halogen, cyano, nitro, hydroxyl, $C_1$-$C_{12}$ alkoxy, $C_1$-$C_{12}$ alkoxycarbonyl, $C_1$-$C_{12}$ alkoxycarbonyloxy, $C_1$-$C_{12}$ alkylacyloxy, carboxyl, sulfhydryl, $C_1$-$C_{12}$ alkylthio, or $NR_4R_4'$; $R_3$, $R_3'$, $R_4$, and $R_4'$ are each independently selected from the group consisting of hydrogen, $C_1$-$C_{12}$ alkyl, $C_2$-$C_{12}$ alkenyl, $C_2$-$C_{12}$ alkynyl, $C_3$-$C_{12}$ cycloalkyl, $C_6$-$C_{14}$ aryl, heteroaryl, and heterocyclyl, or $R_3$ and $R_3'$, $R_4$ and $R_4'$ together with the nitrogen atoms to which they are attached form heterocyclyl.

In one embodiment of the present disclosure, in the compound having the structure as represented by formula (I), or the stereoisomer, the tautomer, the isotope-enriched analog, the solvate, and the pharmaceutically acceptable salt thereof provided in the present disclosure, $R_1$ is selected from the group consisting of hydrogen and $C_1$-$C_{12}$ alkyl; preferably, $R_1$ is selected from the group consisting of hydrogen and $C_1$-$C_6$ alkyl; more preferably, $R_1$ is selected from the group consisting of hydrogen and methyl; most preferably, $R_1$ is selected from hydrogen.

In one embodiment of the present disclosure, in the compound having the structure as represented by formula (I), or the stereoisomer, the tautomer, the isotope-enriched analog, the solvate, and the pharmaceutically acceptable salt

thereof provided in the present disclosure, $R_2$ is selected from the group consisting of R-(C=O)-, R-(C=S)-, R-(S=O)-, R-($SO_2$)-, R-CH(OH)-, R-O-, R-S-, and $NO_2$, where R is selected from the group consisting of hydrogen, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, heteroaryl, heterocyclyl, and $NR_3R_3$', where the alkyl, alkenyl, alkynyl, cycloalkyl, aryl, heteroaryl, and heterocyclyl are optionally substituted with halogen, cyano, nitro, hydroxyl, $C_1$-$C_6$ alkoxy, $C_1$-$C_6$ alkoxycarbonyl, $C_1$-$C_6$ alkoxycarbonyloxy, $C_1$-$C_6$ alkylacyloxy, carboxyl, sulfhydryl, $C_1$-$C_6$ alkylthio, or $NR_4R_4$'; $R_3$, $R_3$', $R_4$, and $R_4$' are each independently selected from the group consisting of hydrogen, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, heteroaryl, and heterocyclyl, or $R_3$ and $R_3$', $R_4$ and $R_4$' together with the nitrogen atoms to which they are attached form heterocyclyl.

In a preferred embodiment of the present disclosure, in the compound having the structure as represented by formula (I), or the stereoisomer, the tautomer, the isotope-enriched analog, the solvate, and the pharmaceutically acceptable salt thereof provided in the present disclosure, $R_2$ is selected from the group consisting of R-(C=O)-, R-(C=S)-, R-(S=O)-, R-($SO_2$)-, R-CH(OH)-, R-O-, R-S-, and $NO_2$, where R is selected from the group consisting of hydrogen, $C_1$-$C_4$ alkyl, $C_2$-$C_4$ alkenyl, $C_2$-$C_4$ alkynyl, $C_3$-$C_6$ cycloalkyl, phenyl, heteroaryl, heterocyclyl, and $NR_3R_3$', where the alkyl, alkenyl, alkynyl, cycloalkyl, phenyl, heteroaryl, and heterocyclyl are optionally substituted with halogen, cyano, nitro, hydroxyl, $C_1$-$C_4$ alkoxy, $C_1$-$C_4$ alkoxycarbonyl, $C_1$-$C_4$ alkoxycarbonyloxy, $C_1$-$C_4$ alkylacyloxy, carboxyl, sulfhydryl, $C_1$-$C_4$ alkylthio, or $NR_4R_4$'; $R_3$, $R_3$', $R_4$, and $R_4$' are each independently selected from the group consisting of hydrogen, $C_1$-$C_4$ alkyl, $C_2$-$C_4$ alkenyl, $C_2$-$C_4$ alkynyl, $C_3$-$C_6$ cycloalkyl, phenyl, heteroaryl, and heterocyclyl, or $R_3$ and $R_3$', $R_4$ and $R_4$' together with the nitrogen atoms to which they are attached form heterocyclyl.

In a preferred embodiment of the present disclosure, in the compound having the structure as represented by formula (I), or the stereoisomer, the tautomer, the isotope-enriched analog, the solvate and the pharmaceutically acceptable salt thereof provided in the present disclosure, $R_1$ is selected from the group consisting of hydrogen and $C_1$-$C_6$ alkyl; $R_2$ is selected from the group consisting of R-(C=O)-, R-(C=S)-, R-(S=O)-, R-($SO_2$)-, R-CH(OH)-, R-O-, R-S-, and $NO_2$, where R is selected from the group consisting of hydrogen, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, heteroaryl, heterocyclyl, and $NR_3R_3$', where the alkyl, alkenyl, alkynyl, cycloalkyl, aryl, heteroaryl, and heterocyclyl are optionally substituted with halogen, cyano, nitro, hydroxyl, $C_1$-$C_6$ alkoxy, $C_1$-$C_6$ alkoxycarbonyl, $C_1$-$C_6$ alkoxycarbonyloxy, $C_1$-$C_6$ alkylacyloxy, carboxyl, sulfhydryl, $C_1$-$C_6$ alkylthio, or $NR_4R_4$'; $R_3$, $R_3$', $R_4$, and $R_4$' are each independently selected from the group consisting of hydrogen, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, heteroaryl, and heterocyclyl, or $R_3$ and $R_3$', $R_4$ and $R_4$' together with the nitrogen atoms to which they are attached form heterocyclyl.

In a preferred embodiment, the present disclosure provides the compound or the stereoisomer, the tautomer, the isotope-enriched analog, the solvate and the pharmaceutically acceptable salt thereof, wherein the compound has a structure as represented by formula (II),

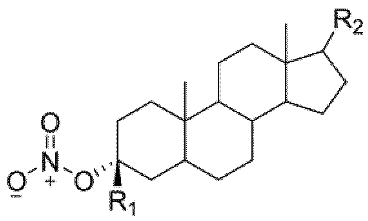

(II);

and

where $R_1$, and $R_2$ are as defined herein.

In a preferred embodiment, the present disclosure provides the compound or the stereoisomer, the tautomer, the isotope-enriched analog, the solvate and the pharmaceutically acceptable salt thereof, wherein the compound has a structure as represented by formula (III),

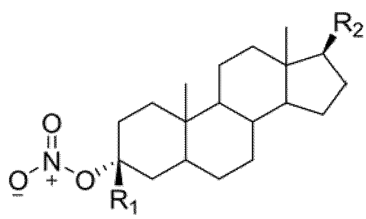

(III);

and

where $R_1$, and $R_2$ are as defined herein.

In a preferred embodiment, the present disclosure provides the compound or the stereoisomer, the tautomer, the isotope-enriched analog, the solvate and the pharmaceutically acceptable salt thereof, wherein the compound has a structure as represented by formula (IV),

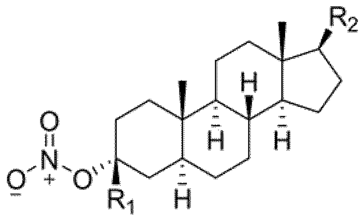

(IV);

and

where $R_1$, and $R_2$ are as defined herein.

In a preferred embodiment, the present disclosure provides the compound or the stereoisomer, the tautomer, the isotope-enriched analog, the solvate and the pharmaceutically acceptable salt thereof, wherein the compound has a structure as represented by the following formula,

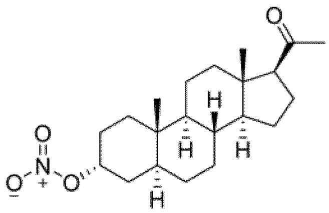

.

## Pharmaceutical composition and administration

The compound or the pharmaceutically acceptable salt, the stereoisomer, the tautomer, the prodrug, and the solvate thereof provided in the present disclosure is typically administered in a form of a pharmaceutical composition. The present disclosure thus provides a pharmaceutical composition comprising the compound provided in the present disclosure as an active ingredient, and one or more pharmaceutically acceptable carriers. The pharmaceutical composition may be administered alone or in combination with other therapeutic agents. Such compositions are prepared in a manner well known in the art (e.g., Reminton's Pharmaceutical Sciences, Mace Publishing Co., Philadelphia, PA 17th Ed.(1985); and Modern Pharmaceutics, Marcel Dekker, Inc.3rd Ed. (G.S.Banker & C.T.Rhodes, Eds.)).

Pharmaceutically acceptable carriers may be solid or liquid, where solid carriers may be one or more substances serving as excipients, diluents, sweeteners, solubilizers, lubricants, binders, tablet disintegrants, stabilizers, preservatives, or encapsulating materials, and liquid carriers may be solvents or liquid dispersion media. Suitable solid carriers include, but are not limited to, for example, cellulose, glucose, lactose, mannitol, magnesium stearate, magnesium carbonate, sodium carbonate, sodium saccharin, sucrose, dextrin, talc, starch, pectin, gelatin, tragacanth gum, acacia gum, sodium alginate, methylparaben, methylcellulose, sodium carboxymethylcellulose, low-melting point wax, and cocoa butter. Suitable liquid carriers include, but are not limited to, water, ethanol, polyols (e.g., glycerol, propylene glycol, and liquid polyethylene glycol), vegetable oils (e.g., peanut oil, cottonseed oil, safflower oil, sesame oil, olive oil, corn oil, and soybean oil), glycerides, agar, pyrogen-free water, isotonic saline, Ringer's solution, and mixtures thereof.

The pharmaceutical composition of the present disclosure may be in any form suitable for an intended administration method. For example, when intended for oral use, the pharmaceutical composition can be prepared into a tablet, a lozenge, a buccal tablet, an aqueous or oily suspension, a dispersible powder or granule, an emulsion, a hard or soft capsule, a syrup or an elixir, a solution, a spray. The composition intended for oral administration can be prepared according to any method known in the art for the manufacture of pharmaceutical compositions. The pharmaceutical composition of the present disclosure may be in the form of a sterile injectable preparation, such as a sterile injectable aqueous or oily suspension. The pharmaceutical composition of the present disclosure may also be prepared as a preparation suitable for intrapulmonary or intranasal administration, such as a preparation for aerosol or dry powder administration, a nasal drop or a nasal spray. The pharmaceutical composition of the present disclosure may also be prepared as a suppository suitable for rectal administration. The pharmaceutical composition of the present disclosure may also be prepared as a transdermal preparation for topical administration, or as an eye drop suitable for ocular administration.

The pharmaceutical compositions of the present disclosure may be administered via arterial injection, intravenous injection, intraperitoneal injection, parenteral injection, intramuscular injection, subcutaneous injection, sublingual administration, oral administration, topical single-dose or multiple-dose administration.

The effective dose of the compound of the present disclosure depends at least on the nature of the condition being treated, toxicity, method of delivery, and pharmaceutical preparation, and will be determined by clinicians using conventional dose escalation studies. It can be expected that the effective dose is about 0.0001 mg to about 100 mg per kilogram of body weight per day; typically about 0.01 mg to about 10 mg per kilogram of body weight per day; more typically, about 0.01 mg to about 5 mg per kilogram of body weight per day; most typically, about 0.05 mg to about 0.5 mg per kilogram of body weight per day. For example, a candidate daily dose for an adult weighing about 70 kg will be within the range of 1 mg to 1000 mg, preferably within the range of 5 mg to 500 mg, and may be administered in the form of a single-dose or multiple-dose.

## Indication

The compound or the stereoisomer, the tautomer, the isotope-enriched analog, and the solvate thereof of the present disclosure can be used for preventing and/or treating GABA-A receptor mediated diseases. In particular, the compound or the stereoisomer, the tautomer, the isotope-enriched analog, and the solvate thereof of the present disclosure can be used for preventing and/or treating central nervous system diseases. In particular, the compound or the stereoisomer, the tautomer, the isotope-enriched analog, and the solvate thereof of the present disclosure can be used for preventing and/or treating diseases including, but not limited to, post-natal depression, depression, major depressive disorder, bipolar affective disorder, mood disorder, anxiety disorder, post-traumatic stress disorder (PTSD), premenstrual dysphoric disorder (PMDD), premenstrual syndrome, generalized anxiety disorder, seasonal affective disorder (SAD), social anxiety disorder, memory loss, stress intolerance, type C Niemann-Pick's disease or related nerves or physical symptoms, epilepsy, essential tremor, epileptiform disorder, NMDA hypofunction, migraine, status epilepticus, sleep disorder, fragile X syndrome, 5α-reductase inhibitor-induced depression, PCDH19 female pediatric epilepsy, sexual dysfunction, cognitive disorder, Parkinson's disease and Alzheimer's disease.

## Combined-medication

The compound of the present disclosure may be administered as a monotherapeutic agent for treating and/or preventing of GABA-A receptor mediated diseases, or central nervous system diseases, or diseases selected from the group consisting of the group consisting of post-natal depression, depression, major depressive disorder, bipolar affective disorder, mood disorder, anxiety disorder, post-traumatic stress disorder (PTSD), premenstrual dysphoric disorder (PMDD), premenstrual syndrome, generalized anxiety disorder, seasonal affective disorder (SAD), social anxiety disorder, memory loss, stress intolerance, type C Niemann-Pick's disease or related nerves or physical symptoms, epilepsy, essential tremor, epileptiform disorder, NMDA hypofunction, migraine, status epilepticus, sleep disorder, fragile X syndrome, 5α-reductase inhibitor-induced depression, PCDH19 female pediatric epilepsy, sexual dysfunction, Parkinson's disease and Alzheimer's disease. The compound of the present disclosure may also be administered in combination with one or more additional therapeutic agents to treat the diseases mentioned herein. The one or more additional therapeutic agents include, but are not limited to, neuroactive steroids, such as pregnanolone, allopregnanolone, alfadolone, ganaxolone, alfaxalone; benzodiazepines; barbiturates.

## Treatment method and use

In one embodiment, the present disclosure provides a method for treating and/or preventing GABA-A receptor mediated diseases, and the method includes administering to an individual in need the compound or the stereoisomer, the tautomer, the isotope-enriched analog, and the solvate thereof, or the pharmaceutical composition containing same of the present disclosure in a therapeutically effective amount. In a further embodiment, the present disclosure provides a method for treating and/or preventing central nervous system diseases, which method includes administering to an individual in need the compound or the stereoisomer, the tautomer, the isotope-enriched analog, and the solvate thereof, or the pharmaceutical composition containing same of the present disclosure in a therapeutically effective amount. In a further embodiment, the present disclosure provides a method for treating and/or preventing diseases including post-natal depression, depression, major depressive disorder, bipolar affective disorder, mood disorder, anxiety disorder, post-traumatic stress disorder (PTSD), premenstrual dysphoric disorder (PMDD), premenstrual syndrome, generalized anxiety disorder, seasonal affective disorder (SAD), social anxiety disorder, memory loss, stress intolerance, type C Niemann-Pick's disease or related nerves or physical symptoms, epilepsy, essential tremor, epileptiform disorder, NMDA hypofunction, migraine, status epilepticus, sleep disorder, fragile X syndrome, 5α-reductase inhibitor-induced depression, PCDH19 female pediatric epilepsy, sexual dysfunction, cognitive disorder, Parkinson's disease and Alzheimer's disease, and the method includes administering to an individual in need the compound or the stereoisomer, the tautomer,

the isotope-enriched analog, the solvate, and the pharmaceutically acceptable salt thereof, or the pharmaceutical composition containing same of the present disclosure in a therapeutically effective amount.

The present disclosure provides use of the compound or the stereoisomer, the tautomer, the isotope-enriched analog, and the solvate thereof of the present disclosure as an active therapeutic substance in manufacture of a medicament for treating and/or preventing GABA-A receptor-mediated diseases. More particularly, the present disclosure provides use of the compound or the stereoisomer, the tautomer, the isotope-enriched analog, and the solvate thereof of the present disclosure as the active therapeutic substance in manufacture of the medicament for treating and/or preventing central nervous system diseases. More particularly, the present disclosure provides use of the compound or the stereoisomer, the tautomer, the isotope-enriched analog, and the solvate thereof of the present disclosure as the active therapeutic substance in manufacture of the medicament for treating and/or preventing diseases including, but not limited to, post-natal depression, depression, major depressive disorder, bipolar affective disorder, mood disorder, anxiety disorder, post-traumatic stress disorder (PTSD), premenstrual dysphoric disorder (PMDD), premenstrual syndrome, generalized anxiety disorder, seasonal affective disorder (SAD), social anxiety disorder, memory loss, stress intolerance, type C Niemann-Pick's disease or related nerves or physical symptoms, epilepsy, essential tremor, epileptiform disorder, NMDA hypofunction, migraine, status epilepticus, sleep disorder, fragile X syndrome, 5$\alpha$-reductase inhibitor-induced depression, PCDH19 female pediatric epilepsy, sexual dysfunction, cognitive disorder, Parkinson's disease and Alzheimer's disease.

## General synthetic methods

The compound of the present disclosure can be prepared using the methods disclosed herein, modified approaches thereof, and methods well known in the art. Typical embodiments of the compound of the present disclosure can be synthesized using the following general reaction scheme. It is apparent from the description herein that correspondingly different products can be obtained by using other materials having similar structures in place of the reaction raw materials. Reaction raw materials are typically obtained from commercial sources or synthesized using publicly available methods.

Reaction scheme

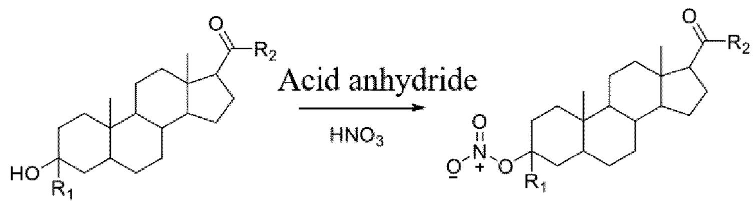


I-a I

The compound as represented by formula (I-a) is reacted with an acid anhydride compound (e.g., acetic anhydride, and trifluoroacetic anhydride) and nitric acid in a suitable solvent to obtain the compound as represented by formula (I).

The following examples are provided to illustrate the preparation of the compound of the present disclosure but not to limit the present disclosure in any way.

## Example

### Example 1 Synthesis of compound 1

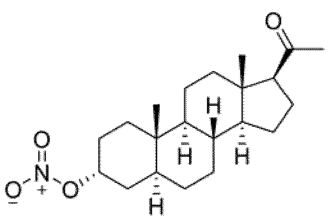

(Compound 1)

5.00 g of allopregnanolone and 105.0 mL of dichloromethane were added to a 250 mL three-neck flask, then stirred and dissolved, and then cooled to below -10°C. 45.0 mL of acetic anhydride was added dropwise to the three-neck flask. 10 mL of nitric acid was slowly added dropwise thereto below -10°C, and after the addition was completed, a reaction was conducted continuously below -10°C until completion. A resulting reaction solution was introduced into 300 mL of water, and stirred. A resulting organic phase was washed with 200 mL of water twice, and distilled under reduced pressure. A resulting residue was separated by column chromatography to obtain an off-white solid (4.86 g, 85% yield).

$^1$H NMR (400 MHz, chloroform-d) δ 2.53 (t, J = 9.0 Hz, 1H), 2.39 (td, J = 14.5, 13.6, 6.5 Hz, 1H), 2.34 - 2.26 (m, 2H), 2.25 - 2.15 (m, 1H), 2.12 (s, 4H), 2.04 (dtt, J = 12.7, 6.2, 3.2 Hz, 3H), 1.76 - 1.61 (m, 5H), 1.61 - 1.49 (m, 1H), 1.48 - 1.40 (m, 3H), 1.40 - 1.32 (m, 3H), 1.26 (ddd, J = 23.2, 9.9, 4.4 Hz, 2H), 1.17 (ddd, J = 16.4, 9.5, 4.2 Hz, 2H), 1.01 (s, 3H), 0.94 (ddd, J = 24.6, 12.1, 5.5 Hz, 2H), 0.83 - 0.75 (m, 1H), 0.64 (s, 3H).
MS: m/z$[M+H]^+$ 364.15

**Bioactivity test**

**I. GABA-A receptor positive modulatory activity assay**

Experimental method:

In the experiments, a HEK293 cell line transiently expressing the GABA-A (α4β3δ) receptor was used to investigate the effect of the compound on the GABA-A (α4β3δ) receptor, and the half-maximal effective concentration ($EC_{50}$) value was tested for the compound. In the experiments, a manual patch-clamp system (HEKA EPC 10 signal amplifier and digital switching system) was used for whole-cell current recording of chloride ion currents in the GABA-A channel. All the experiments were conducted at normal room temperature.

When whole-cell sealing was formed, the cell membrane voltage was clamped at -70 mV. In a Gap-free mode, the peak current was recorded after sequentially spraying onto the cell surface a mixture of GABA (from low concentration to high concentration) and the compound of the present disclosure (3 μM). The mode of administration of the test compound was as follows: a mixture of GABA (each concentration) and the compound of the present disclosure (3 μM) was administered 1-2 times, and rinsing was then performed with an extracellular solution for 1 minute before the next concentration was detected. A microelectrode puller was used to pull capillary glass tubes into recording electrodes. The electrode filled with an intracellular solution was loaded into the electrode holder, and under an inverted microscope, the electrode was immersed into the extracellular solution by manipulating a microelectrode manipulator, and the pipette resistance (Rpip) was recorded. The electrode was brought into contact with the cell surface, and negative pressure suction was applied to form a gigaohm seal (GΩ). At this time, fast capacitance compensation was performed, and then the negative pressure was continued to rupture the cell membrane, forming a whole-cell recording mode. Slow capacitance compensation was then performed and experimental parameters such as film capacitance (Cm) and series resistance (Rs) were recorded. No electric leakage compensation was provided.

The coverslip with the cells was placed in a recording bath under the inverted microscope. The blank control (the extracellular solution) and the working solution of the compound of the present disclosure sequentially flowed through the recording bath from low concentration to high concentration by gravity perfusion to act on the cells. During the recording, liquid exchange was performed using a peristaltic pump. Each concentration was independently detected twice using at least two cells.

The GABA-A (α4β3δ) receptor currents following the action of a mixed liquor of GABA (each concentration) and the compound of the present disclosure (3 μM) were normalized to the control current following the action of a mixed liquor of saturated GABA and the compound of the present disclosure (3 μM) ( $\frac{\text{Peak current}_{compound}}{\text{Peak current}_{control}}$ ), and the mean, standard deviation and standard error were calculated for each concentration ratio.

$$Y = \text{Bottom} + (\text{Top}-\text{Bottom})/(1+10\text{^}((\text{LogEC}_{50}-X)\times\text{HillSlope}))$$

$EC_{50}$ value of the compound of the present disclosure was calculated using the above equation, and the dose-dependent effect was subjected to non-linear fitting, where $EC_{50}$ was the half-maximal effective concentration. The calculation of $EC_{50}$ and curve fitting were accomplished using GraphPad Prism software.

Under unchanged experimental protocol and experimental parameters, the above experiment was repeated with allopregnanolone in place of the compound of the present disclosure. The $EC_{50}$ value of allopregnanolone was calculated

using the above equation, and the dose-dependent effect was subjected to non-linear fitting, where $EC_{50}$ was the half-maximal effective concentration. The calculation of $EC_{50}$ and curve fitting were accomplished using GraphPad Prism software.

Experimental results:

The half-maximal effective concentration ($EC_{50}$) value of compound 1 of the present disclosure for the GABA-A receptor is 10.514 nM, and the half-maximal effective concentration ($EC_{50}$) value of allopregnanolone for the GABA-A receptor is 8.328 nM, indicating that compound 1 of the present disclosure has a strong positive modulatory effect on GABA-A receptor, with activity close to that of allopregnanolone (FIG. 1).

**II. *In vitro* metabolic stability experiments**

Experimental background

Cytochrome P450 oxidase (CYP450), also known as hepatic microsomal mixed-function oxidase, is mainly located on the endoplasmic reticulum and mitochondrial inner membranes, and is involved in the metabolism of a variety of endogenous and exogenous substances such as drugs, carcinogens, steroid hormones, and fatty acids. CYP450 oxidoreductase (POR) is the only electron donor for all hepatic microsomal enzymes, and transfers electrons to CYP450 enzymes via reduced nicotinamide adenine dinucleotide phosphate (NADPH). After receiving electrons, the CYP450 enzymes undergo redox reactions with substrates to exert metabolic activity.

Hepatic microsomes contain most phase I enzymes, among which the microsomal mixed-function oxidase system with CYP450 as the main component is the most important. When studying with hepatic microsomes, the addition of corresponding cofactor NADPH can reconstitute an in vitro metabolic system, thereby performing phase I metabolic stability studies by in vitro incubation.

Experimental object

The *in vitro* metabolic stability of compound 1 and allopregnanolone was studied using a commercially available male SD rat liver phase I metabolic stability kit (containing hepatic microsomes and reagents used for the study).

Experimental reagents

Phase I metabolic stability kit (purchased from HUIZHI HEYUAN BIOTECHNOLOGY (BEIJING) CO., LTD), with the following components of the product:

| Name | Specification | Number |
|---|---|---|
| Solution A (20×) | 1 mL/vial | 1 vial |
| Solution B (100×) | 200 μL/vial | 1 vial |
| Hepatic microsomes (20 mg/mL) | 500 μL/vial | 1 vial |
| Positive substrate (testosterone, 10 mM) | 50 μL/vial | 1 vial |
| 0.1 M PBS buffer (pH 7.4) | 12 mL/bottle | 2 bottles |

The components of solution A were: 26.1 mM NADP+, 66 mM glucose 6-phosphate, 66 mM magnesium chloride. The components of solution B were: 40 U/mL glucose 6-phosphate dehydrogenase, 5 mM sodium citrate.

Compounds to be tested: compound 1 solution: dissolved with acetonitrile to prepare a 40 μg/mL solution allopregnanolone solution: dissolved with acetonitrile to prepare a 40 μg/mL solution

Experimental method

Positive substrate testosterone solution (10 mM): diluted with acetonitrile to prepare a 200 μM solution for later use; stop solution & internal standard (testosterone-D3): dissolved with acetonitrile to prepare a 5 ng/mL solution for later use.

1. Experimental group

1) all components of the kit were thawed in an ice bath and placed on ice for later use;
2) an incubation system (taking 200 μL system as an example) was prepared:

| **Preparation of pre-incubation solution A:** | | |
|---|---|---|
| **Component** | **Volume/reaction** | **Remarks** |
| Solution A of the kit | 10 μL | Mix uniformly, pre-incubate at 37°C for **5 min,** subpackage at **40 μL**/tube for later use. |
| Solution B of the kit | 2 μL | |
| 0.1 M PBS buffer | 28 μL | |
| **Preparation of pre-incubation solution B:** | | |
| **Component** | **Volume/reaction** | **Remarks** |
| 0.1 M PBS buffer | 154 μL | Prepare just before needed. The amount of acetonitrile added to the system must not exceed 1%. |
| Compound to be tested | 1 μL | |
| Hepatic microsomes | 5 μL | |

3) 160 μL of pre-incubation solution B was added into the centrifuge tube containing 40 μL of pre-incubation solution A, uniform mixing was performed by pipetting three times, and the resulting mixture was immediately placed in a water bath at 37°C for incubation and timing was started;
4) at predetermined incubation time points (0, 5, 10, 15, 30, 45, 60 minutes), the incubation solution was quantitatively taken out, and 200 μL of stop solution (5 ng/mL testosterone-D3 in acetonitrile, pre-chilled at 4°C) was added to terminate the reaction (stop solution volume: incubation system volume = 1: 1). Experimental samples were prepared in triplicate at each time point.

2. Positive control group

To verify the validity of the experiment, a positive control group was established concurrently in the whole experiment to evaluate whether the incubation system functioned properly, with the preparation method as follows: the compound to be tested was replaced with the positive substrate testosterone (200 μM in acetonitrile) from the phase I metabolic stability kit, and the other components of the incubation system and experimental method were the same as those of the experimental group.

3. Data processing

The ratio of the chromatographic peak area of the compound to be tested to the chromatographic peak area of the testosterone-D3 (internal standard) was used as the calculated data. By taking the peak area ratio at time zero of the test compound as 100%, the remaining percentages were calculated by comparing the peak area ratio at each time point with the peak area ratio at time zero, and the natural logarithms of the remaining percentages of the substrate at each time point are then subjected to linear regression against the incubation times to obtain the slope k. Therefore, the in vitro metabolic half-life ($T_{1/2}$) and intrinsic clearance ($CL_{int}$) were calculated using the following formulas:

$$\text{Elimination half-life} = \frac{-0.693}{k}$$

$$\text{Hepatic microsomal intrinsic clearance} = \frac{0.693}{\text{Elimination half-life (min)}} \times \frac{\text{Incubation volume (mL)}}{\text{Hepatic microsome mass (mg)}}$$

The detection results were as follows:

| | Peak area ratio (test compound/testosterone-D3 (internal standard)) | | | | | | |
|---|---|---|---|---|---|---|---|
| Time (min) | 0 | 5 | 10 | 20 | 30 | 45 | 60 |
| Positive control-1 | 20.12 | - | 0.05 | - | - | - | - |
| Positive control-2 | 19.58 | - | 0.08 | - | - | - | - |
| Positive control-3 | 20.33 | - | 0.06 | - | - | - | - |
| Positive control mean value | 20.01 | - | 0.06 | - | - | - | - |
| Compound 1-1 | 2.26 | 1.68 | 2.01 | 1.84 | 1.72 | 1.39 | 1.52 |
| Compound 1-2 | 2.51 | 2.21 | 2.06 | 1.99 | 1.85 | 1.67 | 1.59 |
| Compound 1-3 | 2.36 | 2.69 | 2.56 | 2.14 | 1.65 | 1.58 | 1.65 |
| Compound 1 mean value | 2.38 | 2.19 | 2.21 | 1.99 | 1.74 | 1.55 | 1.59 |
| Allopregnanolone-1 | 23.3 | 2.72 | 0.19 | 0.04 | Not detected | Not detected | Not detected |
| Allopregnanolone-2 | 26.2 | 3.21 | 0.3 | 0.06 | Not detected | Not detected | Not detected |
| Allopregnanolone-3 | 24.1 | 2.23 | 0.15 | 0.02 | Not detected | Not detected | Not detected |
| Allopregnanolone mean value | 24.55 | 2.72 | 0.21 | 0.04 | Not detected | Not detected | Not detected |
| "-" represents not detected. | | | | | | | |

Half-life and intrinsic clearance calculation results

| **Compound** | **$T_{1/2}$ (min)** | **k** | **In vitro $CL_{int}$ (μL/min/mg protein)** |
|---|---|---|---|
| Compound 1 | 93.54 | -0.0074 | 14.82 |
| Allopregnanolone | 2.17 | -0.3196 | 639.28 |

Experimental results show that the positive control testosterone is substantially completely metabolized within 10 minutes, indicating that the incubation system functions properly. Allopregnanolone is metabolized extremely rapidly in the hepatic microsomes *in vitro,* being basically completely metabolized within 10 minutes, with a metabolic half-life of 2.17 min and an intrinsic clearance of 639.28 μL/min/mg, while compound 1 of the present disclosure is metabolized more slowly, with a metabolic half-life of 93.54 min and an intrinsic clearance of 14.82 μL/min/mg.

**III. Pharmacokinetic analysis of tail vein administration in mice**

Experimental drugs

Allopregnanolone: 0.5 mg/mL aqueous solution (solubilized with 30% cyclodextrin)
Compound 1: 0.5 mg/mL aqueous solution (solubilized with 30% cyclodextrin)

Experimental animals

Female ICR mice (body weight: 20 g to 22 g) were purchased from BEIJING HFK BIOSCIENCE CO., LTD.

Experimental method

1. Tail vein administration and treatment in mice:

60 female ICR mice were divided into two groups A and B, with 30 mice per group (5 mice per blood collection time point), and allopregnanolone and compound 1 were administered via tail vein injection at a dose of 5 mg/kg to both groups A and B, respectively. Blood was collected via eyeball extraction at 5 min, 30 min, 1 h, 5 h, 8 h, and 12 h after administration, respectively, anticoagulated with heparin, and centrifuged, and then the plasma was separated and stored at -80°C for later testing. The animals were sacrificed at blood collection time points (5 min, 1 h and 8 h) for brain tissue collection, and

the collected brain tissues were stored at -80°C for later testing.

2. Biological sample treatment

(1) Plasma sample

100 μL of plasma sample, 10 μL of internal standard solution (100 ng/mL testosterone D-3 solution), and 310 μL of acetonitrile were accurately measured, uniformly mixed by vortexing, and centrifuged (14000 rpm, 4°C), and a resulting supernatant was taken to obtain the plasma sample.

(2) Brain tissue sample

About 100 mg of brain tissue sample was taken, and accurately weighed, 100 μL of water and 400 μL acetonitrile were accurately added thereto, and a resulting mixture was subjected to homogenization. 100 μL of brain tissue homogenate, 10 μL of internal standard solution (100 ng/mL testosterone D-3 solution), and 310 μL of acetonitrile were accurately measured, uniformly mixed by vortexing, and centrifuged (14000 rpm, 4°C), and a resulting supernatant was taken to obtain the brain tissue sample.

3. Biological sample assay

(1) Preparation of a series of standard solutions

① Plasma matrix standard solution

Appropriate amounts of allopregnanolone and compound 1 were separately taken and quantitatively diluted by adding acetonitrile to prepare mixed standard solutions containing 5000 ng, 2000 ng, 1000 ng, 500 ng, 200 ng, 100 ng, 50 ng, 20 ng of each of allopregnanolone and compound 1 per mL. 100 μL of blank plasma sample, 10 μL of mixed standard solution, 10 μL of internal standard solution (100 ng/mL testosterone D-3 solution), and 300 μL of acetonitrile were accurately measured, uniformly mixed by vortexing, and centrifuged (14000 rpm, 4°C), and a resultingsupernatant was taken to obtain the plasma matrix standard solution.

② Brain tissue matrix standard solution

Appropriate amounts of allopregnanolone and compound 1 were separately taken and quantitatively diluted by adding acetonitrile to prepare mixed standard solutions containing 5000 ng, 2000 ng, 1000 ng, 500 ng, 200 ng, 100 ng, 50 ng of each of allopregnanolone and compound 1 per 1 mL. About 100 mg of blank brain tissue sample was taken, and accurately weighed, 100 μL of water and 400 μL acetonitrile were accurately added thereto, and a resulting mixture was subjected to homogenization using a homogenizer. 100 μL of brain tissue homogenate, 10 μL of mixed standard solution, 10 μL of internal standard solution (100 ng/mL testosterone D-3 solution) and 300 μL of acetonitrile were accurately measured, uniformly mixed by vortexing, and centrifuged (14000 rpm, 4°C), and a resulting supernatant was taken to obtain the Brain tissue matrix standard solution.

(2) Analysis method

The UPLC-MS/MS method with testosterone D-3 as the internal standard was used to perform analysis and determination by the standard curve method.
The UPLC parameters were as follows:

| | |
|---|---|
| Chromatographic column | Waters ACQUITY UPLC BEH C18 1.7 μm 2.1×50 mm |
| Column temperature | 40°C |
| Flow rate | 0.5 ml/min |
| Injection volume | 5 μl |
| Mobile phase A | Water-acetonitrile-formic acid (95:5:0.1) |
| Mobile phase B | Acetonitrile-formic acid (100:0.1) |

(continued)

| | Time (min) | A (%) | B (%) |
|---|---|---|---|
| Gradient | 0 | 90 | 10 |
| | 0.2 | 90 | 10 |
| | 6.0 | 15 | 85 |
| | 7.0 | 15 | 85 |
| | 7.01 | 90 | 10 |
| | 10 | 90 | 10 |
| MS parameters | | | |

| Detection mode | MRM positive ion mode | | | | |
|---|---|---|---|---|---|
| IS | 5500 | | | | |
| CUR | 35 | | | | |
| GS1 | 90 | | | | |
| GS2 | 55 | | | | |
| TEM | 550°C | | | | |
| Parameters | Q1 | Q2 | CE | DP | Dwell Time |
| Allopregnanolone | 319.2 | 283.3※ | 18 | 100 | 200 |
| | | 301.3 | 15 | 100 | 200 |
| | | 257.2 | 27 | 100 | 200 |
| Compound 1 | 364.2 | 283.3※ | 14 | 100 | 200 |
| | | 346.5 | 14 | 100 | 200 |
| Testosterone D-3 (internal standard) | 292.2 | 109.2 | 30 | 80 | 200 |
| | | 97.0※ | 28 | 60 | 200 |
| Note: ※ is the quantitative ion. | | | | | |

4. Experimental results

| Compound | Compound 1 | Allopregnanolone |
|---|---|---|
| Clearance (L/h/kg) | 11.8 | 13.1 |
| $T_{1/2}$ (h) | 0.84 | 0.68 |
| Brain/plasma ratio (5 min) | 0.65 | 1.28 |
| Brain/plasma ratio (1 h) | 7.58 | 2.71 |
| Brain/plasma ratio (8 h) | 2.27 (low level in brain tissue, extremely low level in blood) | Extremely low level in brain tissue, undetectable in blood |

Both Compound 1 and allopregnanolone can rapidly cross the blood-brain barrier after tail vein administration, thereby entering the brain to exert pharmacological effects; while the clearance rate of compound 1 in the brain is much slower than that of allopregnanolone; and the brain tissue concentrations (ng/g) at 5 min, 1 h and 8 h after administration of compound 1 are 1138.9, 652.0, 31.0, respectively, while the brain tissue concentration (ng/g) at 5 min, 1 h and 8 h after administration of allopregnanolone are 1073.2, 47.3, 14.4, respectively. This indicates that compound 1 has more stable

metabolism in brain tissue.

**IV. Pharmacokinetic analysis of tail vein administration and oral administration in female rats**

Experimental drugs

Tail vein injection:

Allopregnanolone: 0.5 mg/mL aqueous solution (solubilized with 30% cyclodextrin, abbreviated as: $A_{intravenous}$)
Compound 1: 0.5 mg/mL aqueous solution (solubilized with 30% cyclodextrin, abbreviated as: $B_{intravenous}$)

Oral gavage administration:

Allopregnanolone: 1.5 mg/mL oil solution (using sesame oil as solvent, abbreviated as: $A_{oral}$)
Compound 1: 1.5 mg/mL oil solution (using sesame oil as solvent, abbreviated as: $B_{oral}$)

Experimental animals

Female SD rats (body weight: 200 g to 220 g) were purchased from BEIJING HFK BIOSCIENCE CO., LTD.

Experimental method

1. Tail vein administration and treatment in rats:

60 female SD rats were divided into two groups ($A_{intravenous}$ and $B_{intravenous}$), with 30 rats per group (5 rats per blood collection time point). The drugs $A_{intravenous}$ and $B_{intravenous}$ were administered via tail vein injection at 3 mg/kg each (about 1.2 mL of drug solution). Blood was collected from the orbital venous plexus at time points (5 min, 30 min, 1 h, 2 h, 4 h, and 8 h) after administration, respectively, anticoagulated with heparin, and centrifuged, and then the plasma was separated and stored at -80°C for later testing. At 8 h after blood collection, the animals were sacrificed, and the brain and liver tissues were taken and stored at -80°C for later testing.

2. Oral gavage administration and treatment in rats:

60 female SD rats were divided into two groups ($A_{oral}$ and $B_{oral}$), with 30 rats per group (5 rats per blood collection time point). The drugs $A_{oral}$ and $B_{oral}$ were administered via oral gavage administration at 12 mg/kg each (about 1.6 mL of drug solution). Blood was collected from the orbital venous plexus at each time point (5 min, 30 min, 1 h, 2 h, 4 h, and 8 h) after administration, respectively, anticoagulated with heparin, and centrifuged, and then the plasma was separated and stored at -80°C for later testing. At 8 h after blood collection, the animals were sacrificed, and the brain and Liver tissues were taken and stored at -80°C for later testing.

3. Sample treatment

1) Plasma sample

100 μL of plasma sample, 10 μL of internal standard solution (100 ng/mL testosterone D-3 solution), and 310 μL of acetonitrile were accurately measured, uniformly mixed by vortexing, and centrifuged (14000 rpm, 4°C), and a resulting supernatant was taken and detected using UPLC-MS/MS (the analysis method was the same as that in pharmacokinetic analysis in mice).

2) Brain and liver tissue samples

About 100 mg of brain or liver tissue sample was taken, and accurately weighed. 100 μL of water and 400 μL acetonitrile were accurately added thereto, and a resulting mixture was subjected to homogenization. 100 μL of brain or liver tissue homogenate, 10 μL of internal standard solution (100 ng/mL testosterone D-3 solution), and 310 μL of acetonitrile were accurately measured, uniformly mixed by vortexing, and centrifuged (14000 rpm, 4°C), and a resulting supernatant was taken and detected using UPLC-MS/MS (the analysis method was the same as that in pharmacokinetic analysis in mice).

4. Experimental results

Pharmacokinetic parameters after tail vein administration to rats

| Compound | Compound 1 | Allopregnanolone |
|---|---|---|
| Clearance (L/h/kg) | 5.27 | 6.53 |
| $T_{1/2}$ (h) | 0.96 | 0.87 |
| Brain/plasma ratio (8 h) | 6.68 | Undetectable in both brain tissue and plasma |

Pharmacokinetic parameters after oral administration to rats

| Compound | Compound 1 | Allopregnanolone |
|---|---|---|
| Clearance (L/h/kg) | 0.58 | / |
| Oral bioavailability (%) | >65 | 0 |
| Brain/plasma ratio (8 h) | 2.95 | Undetectable in both brain tissue and plasma |

After tail vein administration of allopregnanolone and compound 1 to rats, respectively, allopregnanolone is undetectable in both brain tissue and plasma at 8 h after administration in both groups, while the concentration of compound 1 in brain tissue remains relatively high (209.1 ng/g) in the compound 1 administration group, with a brain/plasma ratio of 6.68, indicating that compound 1 has a much slower clearance rate in the brain than allopregnanolone, and exhibits stable metabolism in the brain tissue.

After oral gavage administration of allopregnanolone and compound 1 to rats, respectively, allopregnanolone is undetectable in both plasma and brain tissue of animals in the allopregnanolone group at all time points, indicating that allopregnanolone is essentially not orally administrable. In the compound 1 group, after oral administration, the drug concentration in the plasma continues to increase, and $C_{max}$ is still undetected at 8 h, indicating that compound 1 is readily absorbed into the blood and has a low clearance rate, with $T_{max}$ > 8 h and oral bioavailability > 65%; and after oral absorption into the blood, compound 1 readily crosses the blood-brain barrier, maintaining a very high concentration in the brain tissue (1636.8 ng/g) at 8 h after administration, with a brain/plasma ratio of 2.95, indicating that compound 1 can continuously cross the blood-brain barrier to exert its pharmacological effects after oral administration, and has stable metabolism in the blood.

In addition, the levels of allopregnanolone and compound 1 in the liver tissue after tail vein administration and oral administration are also determined, respectively. The results show that allopregnanolone is undetectable in the liver at 8 h after administration of allopregnanolone via both modes of administration; and at 8 h after oral administration of compound 1, the level of compound 1 in the plasma remains very high (3703.4 ng/g) (allopregnanolone was also detected in the liver tissue (694.2 ng/g)), and at 8 h after intravenous administration of compound 1, a small amount of compound 1 (192.8 ng/g) is detected in the liver, while allopregnanolone is undetectable. The results indicate that compound 1 has a very stable metabolism in the liver.

**V. Pharmacokinetic analysis of oral administration to male rats**

Experimental drugs

Oral gavage administration:

Allopregnanolone: 1.5 mg/mL oil solution (using sesame oil as solvent, abbreviated as: $A_{oral}$)
Compound 1: 1.5 mg/mL oil solution (using sesame oil as solvent, abbreviated as: $B_{oral}$)

Experimental animals

Male SD rats (body weight: 230 g to 250 g) were purchased from BEIJING HFK BIOSCIENCE CO., LTD.

Experimental method

1. Oral gavage administration and treatment in rats:

168 male SD rats were divided into two groups ($A_{oral}$ and $B_{oral}$), with 84 rats per group (7 rats per blood collection time point). The drugs $A_{oral}$ and $B_{oral}$ were administered via oral gavage administration at 12 mg/kg each. Blood was collected from the orbital venous plexus at each time point (30 min, 1 h, 2 h, 3 h, 4 h, 5 h, 6 h, 8 h, 10 h, 12 h, 18 h, and 24 h) after administration, respectively, anticoagulated with heparin, and centrifuged, and then the plasma was separated and stored at -80°C for later testing.

2. Sample treatment

100 μL of plasma sample, 10 μL of internal standard solution (100 ng/mL testosterone D-3 solution), and 310 μL of acetonitrile were accurately measured, uniformly mixed by vortexing, and centrifuged (14000 rpm, 4°C), and a resulting supernatant was taken and detected using UPLC-MS/MS (the analysis method was the same as that in pharmacokinetic analysis in mice).

3. Experimental results

Pharmacokinetic parameters after administration (oral gavage administration) to rats

| Compound 1<br>Blood drug concentration (ng/ml) | 0.5 h | 1 h | 2 h | 3 h | 4 h | 5 h | 6 h | 8 h | 10 h | 12 h | 18 h | 24 h |
|---|---|---|---|---|---|---|---|---|---|---|---|---|
| 1 | 16.2 | 35.9 | 132.2 | 288.6 | 279.6 | 252.4 | 161.2 | 121.7 | 167.3 | 513.5 | 68.5 | 13.7 |
| 2 | 8.7 | 31.5 | 88.8 | 243.6 | 252.2 | 284.1 | 260.7 | 276.1 | 1144.3 | 873.9 | 110.5 | 68.5 |
| 3 | 8.3 | 22.3 | 111.4 | 103.9 | 270.9 | 262.5 | 287.5 | 383.6 | 424.6 | 343.7 | 196.7 | 61.6 |
| 4 | 8.1 | 23.8 | 123 | 130.9 | 343.8 | 410.7 | 409.8 | 523.1 | 914.9 | 730.7 | 97 | 20.7 |
| 5 | 8.4 | 50.6 | 251.1 | 616.7 | 318.5 | 582.4 | 687.1 | 496.4 | 382.7 | 234.9 | 83.3 | 27.6 |
| 6 | 4.1 | 14.1 | 38.2 | 71.9 | 95.1 | 250.4 | 259.8 | 78.9 | 196.1 | 303.8 | 73.4 | 13.8 |
| 7 | 5.7 | 18.9 | 85.8 | 121.8 | 147 | 142.4 | 263.9 | 556.5 | 396.3 | 285.4 | 134.5 | 133.4 |
| Mean value | 8.50 | 28.16 | 118.64 | 225.34 | 243.87 | 312.13 | 332.86 | 348.04 | 518.03 | 469.41 | 109.13 | 48.47 |

| Allopregnanolone Blood drug concentration (ng/ml) | 0.5 h | 1 h | 2 h | 3 h | 4 h | 5 h | 6 h | 8 h | 10 h | 12 h | 18 h | 24 h |
|---|---|---|---|---|---|---|---|---|---|---|---|---|
| 1 | 0 | 0 | 0 | 0 | 0 | 0 | 0 | 0 | 0 | 0 | 0 | 0 |
| 2 | 0 | 0 | 0 | 0 | 0 | 0 | 0 | 0 | 0 | 0 | 0 | 0 |
| 3 | 0 | 0 | 0 | 0 | 0 | 0 | 0 | 0 | 0 | 0 | 0 | 0 |
| 4 | 0 | 0 | 0 | 0 | 0 | 0 | 0 | 0 | 0 | 0 | 0 | 0 |
| 5 | 0 | 0 | 0 | 0 | 0 | 0 | 0 | 0 | 0 | 0 | 0 | 0 |
| 6 | 0 | 0 | 0 | 0 | 0 | 0 | 0 | 0 | 0 | 0 | 0 | 0 |
| 7 | 0 | 0 | 0 | 0 | 0 | 0 | 0 | 0 | 0 | 0 | 0 | 0 |
| Mean value | 0 | 0 | 0 | 0 | 0 | 0 | 0 | 0 | 0 | 0 | 0 | 0 |

| Parameters | Compound 1 | Allopregnanolone |
|---|---|---|
| $AUC_{(0-t)}$ (μg/L*h) | 5834.6 | 0 |
| $AUC_{(0-\infty)}$ (μg/L*h) | 6215.4 | 0 |
| $C_{max}$ (μg/L) | 649.2 | 0 |
| $T_{max}$ (h) | 9.7 | / |
| $t_{1/2}$ (h) | 4.5 | / |

After oral gavage administration of allopregnanolone and compound 1 to male rats, respectively, allopregnanolone is undetectable in the plasma of animals in the allopregnanolone group at all time points, indicating that allopregnanolone is essentially not absorbed into the blood after oral administration. However, in the compound 1 group, after oral administration, the drug concentration in the plasma continues to increase, and $C_{max}$ is reached at about 10 h, indicating that compound 1 is readily absorbed into the blood and has a low clearance rate, with $T_{max}$ of about 10 h and a half-life of 4.5 h.

References mentioned herein are all incorporated herein by reference. It should be understood that variations and modifications may be made to the technical solutions of the present disclosure without departing from the spirit and scope of the present disclosure.

## Claims

1. A compound having a structure as represented by formula (I),

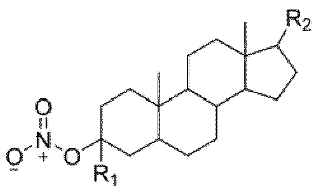

(I)

or a stereoisomer, a tautomer, an isotope-enriched analog, a solvate, and a pharmaceutically acceptable salt thereof, wherein

$R_1$ is selected from the group consisting of hydrogen and $C_1$-$C_{12}$ alkyl; and

$R_2$ is selected from the group consisting of R-(C=O)-, R-(C=S)-, R-(S=O)-, R-($SO_2$)-, R-CH(OH)-, R-O-, R-S-, and $NO_2$, wherein R is selected from the group consisting of hydrogen, $C_1$-$C_{12}$ alkyl, $C_2$-$C_{12}$ alkenyl, $C_2$-$C_{12}$ alkynyl, $C_3$-$C_{12}$ cycloalkyl, $C_6$-$C_{14}$ aryl, heteroaryl, heterocyclyl, and $NR_3R_3$', wherein alkyl, alkenyl, alkynyl, cycloalkyl, aryl, heteroaryl, and heterocyclyl are optionally substituted with halogen, cyano, nitro, hydroxyl, $C_1$-$C_{12}$ alkoxy, $C_1$-$C_{12}$ alkoxycarbonyl, $C_1$-$C_{12}$ alkoxycarbonyloxy, $C_1$-$C_{12}$ alkylacyloxy, carboxyl, sulfhydryl, $C_1$-$C_{12}$ alkylthio, or

$NR_4R_4$'; $R_3$, $R_3$', $R_4$, and $R_4$' are each independently selected from the group consisting of hydrogen, $C_1$-$C_{12}$ alkyl, $C_2$-$C_{12}$ alkenyl, $C_2$-$C_{12}$ alkynyl, $C_3$-$C_{12}$ cycloalkyl, $C_6$-$C_{14}$ aryl, heteroaryl, and heterocyclyl, or $R_3$ and $R_3$', $R_4$ and $R_4$' together with nitrogen atoms to which they are attached form heterocyclyl.

**2.** The compound or the stereoisomer, the tautomer, the isotope-enriched analog, the solvate, and the pharmaceutically acceptable salt thereof of claim 1, wherein

$R_1$ is selected from the group consisting of the hydrogen and $C_1$-$C_6$ alkyl; and
$R_2$ is selected from the group consisting of the R-(C=O)-, the R-(C=S)-, the R-(S=O)-, the R-($SO_2$)-, the R-CH(OH)-, the R-O-, the R-S-, and the $NO_2$, wherein R is selected from the group consisting of the hydrogen, the $C_1$-$C_{12}$ alkyl, the $C_2$-$C_{12}$ alkenyl, the $C_2$-$C_{12}$ alkynyl, the $C_3$-$C_{12}$ cycloalkyl, the $C_6$-$C_{14}$ aryl, the heteroaryl, the heterocyclyl, and the $NR_3R_3$', wherein the alkyl, the alkenyl, the alkynyl, the cycloalkyl, the aryl, the heteroaryl, and the heterocyclyl are optionally substituted with the halogen, the cyano, the nitro, the hydroxyl, the $C_1$-$C_{12}$ alkoxy, the $C_1$-$C_{12}$ alkoxycarbonyl, the $C_1$-$C_{12}$ alkoxycarbonyloxy, the $C_1$-$C_{12}$ alkylacyloxy, the carboxyl, the sulfhydryl, the $C_1$-$C_{12}$ alkylthio, or the $NR_4R_4$'; $R_3$, $R_3$', $R_4$, and $R_4$' are each independently selected from the group consisting of the hydrogen, the $C_1$-$C_{12}$ alkyl, the $C_2$-$C_{12}$ alkenyl, the $C_2$-$C_{12}$ alkynyl, the $C_3$-$C_{12}$ cycloalkyl, the $C_6$-$C_{14}$ aryl, the heteroaryl, and the heterocyclyl, or $R_3$ and $R_3$', $R_4$ and $R_4$' together with the nitrogen atoms to which they are attached form the heterocyclyl.

**3.** The compound or the stereoisomer, the tautomer, the isotope-enriched analog, the solvate, and the pharmaceutically acceptable salt thereof of claim 1, wherein

$R_1$ is selected from the group consisting of the hydrogen and $C_1$-$C_6$ alkyl; and
$R_2$ is selected from the group consisting of the R-(C=O)-, the R-(C=S)-, the R-(S=O)-, the R-($SO_2$)-, the R-CH(OH)-, the R-O-, the R-S-, and the $NO_2$, wherein R is selected from the group consisting of the hydrogen, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, the heteroaryl, the heterocyclyl, and the $NR_3R_3$', wherein the alkyl, the alkenyl, the alkynyl, the cycloalkyl, the aryl, the heteroaryl, and the heterocyclyl are optionally substituted with the halogen, the cyano, the nitro, the hydroxyl, $C_1$-$C_6$ alkoxy, $C_1$-$C_6$ alkoxycarbonyl, $C_1$-$C_6$ alkoxycarbonyloxy, $C_1$-$C_6$ alkylacyloxy, the carboxyl, the sulfhydryl, $C_1$-$C_6$ alkylthio, or the $NR_4R_4$'; $R_3$, $R_3$', $R_4$, and $R_4$' are each independently selected from the group consisting of the hydrogen, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, the heteroaryl, and the heterocyclyl, or $R_3$ and $R_3$', $R_4$ and $R_4$' together with the nitrogen atoms to which they are attached form the heterocyclyl.

**4.** The compound or the stereoisomer, the tautomer, the isotope-enriched analog, the solvate, and the pharmaceutically acceptable salt thereof of any one of claims 1 to 3, wherein the compound is a compound as represented by formula (II):

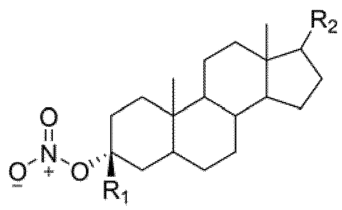
(II);

and
wherein $R_1$ and $R_2$ are as defined in any one of claims 1 to 3.

**5.** The compound or the stereoisomer, the tautomer, the isotope-enriched analog, the solvate, and the pharmaceutically acceptable salt thereof of any one of claims 1 to 3, wherein the compound is a compound as represented by formula (III):

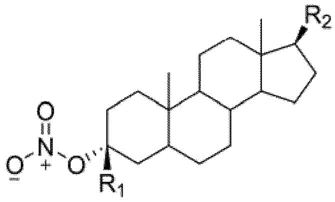

(III);

and
wherein $R_1$ and $R_2$ are as defined in any one of claims 1 to 3.

**6.** The compound or the stereoisomer, the tautomer, the isotope-enriched analog, the solvate, and the pharmaceutically acceptable salt thereof of any one of claims 1 to 3, wherein the compound is a compound as represented by formula (IV):

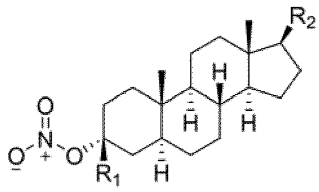

(IV);

and
wherein $R_1$ and $R_2$ are as defined in any one of claims 1 to 3.

**7.** The compound or the stereoisomer, the tautomer, the isotope-enriched analog, the solvate, and the pharmaceutically acceptable salt thereof of any one of claims 1 to 3, wherein the compound is

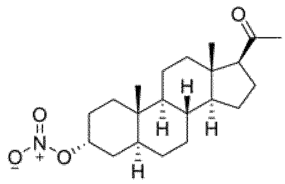

.

**8.** A pharmaceutical composition, comprising the compound or the stereoisomer, the tautomer, the isotope-enriched analog, the solvate, and the pharmaceutically acceptable salt thereof of any one of claims 1 to 7, and a pharmaceutically acceptable carrier.

**9.** Use of the compound of any one of claims 1 to 7 in manufacture of a medicament for treating and/or preventing GABA($\gamma$-aminobutyric acid)-A receptor-mediated diseases.

**10.** The use of claim 9, wherein the GABA-A receptor-mediated diseases comprise post-natal depression, depression, major depressive disorder, bipolar affective disorder, mood disorder, anxiety disorder, post-traumatic stress disorder (PTSD), premenstrual dysphoric disorder (PMDD), premenstrual syndrome, generalized anxiety disorder, seasonal affective disorder (SAD), social anxiety disorder, memory loss, stress intolerance, type C Niemann-Pick's disease or related nerves or physical symptoms, epilepsy, essential tremor, epileptiform disorder, NMDA hypofunction, migraine, status epilepticus, sleep disorder, fragile X syndrome, 5$\alpha$-reductase inhibitor-induced depression, PCDH19 female pediatric epilepsy, sexual dysfunction, cognitive disorder, Parkinson's disease and Alzheimer's disease.

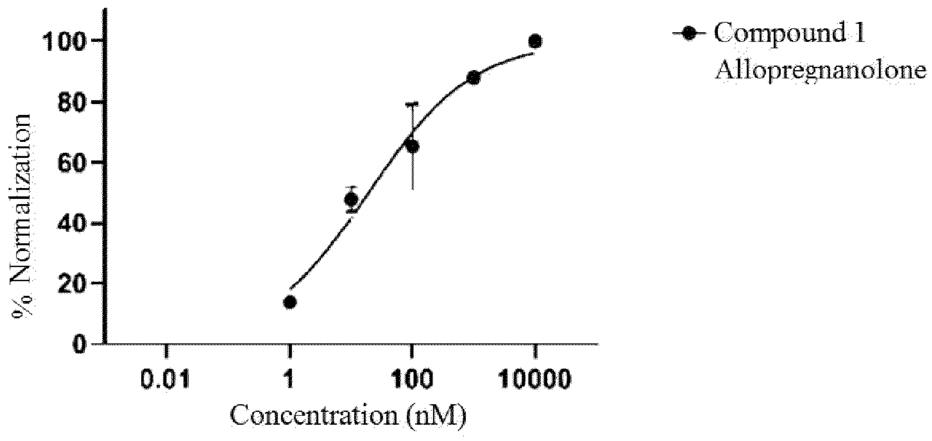


| | Compound 1 3μM | Allopregnanolone3μM |
|---|---|---|
| HillSlope | 0.5627 | 0.2059 |
| EC50 | 10.514 | 8.328 |

**FIG. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 108148106 A
- CN 109503694 A


### Non-patent literature cited in the description


- Reminton's Pharmaceutical Sciences. Mace Publishing Co., 1985
- Modern Pharmaceutics. Marcel Dekker, Inc.